Europäisches Patentamt

European Patent Office

Office européen des brevets

(11) Publication number: **0 346 810**
**A2**

# EUROPEAN PATENT APPLICATION

(21) Application number: **89110621.3**

(51) Int. Cl.⁴: **A61K 31/725**

(22) Date of filing: **12.06.89**

<table>
<tr><td>

The title of the invention has been amended (Guidelines for Examination in the EPO, A-III, 7.3).

(30) Priority: **15.06.88 IL 86753**

(43) Date of publication of application:
**20.12.89 Bulletin 89/51**

(84) Designated Contracting States:
**AT BE CH DE ES FR GB GR IT LI LU NL SE**

</td><td>

(71) Applicant: **YEDA RESEARCH & DEVELOPMENT COMPANY, LIMITED**
**P.O. Box 95**
**Rehovot 76100(IL)**

Applicant: **HADASSAH MEDICAL ORGANIZATION**
**Kiryat Hadassah P.O. Box 12000**
**IL-91120 Jerusalem(IL)**

(72) Inventor: **Cohen, Irun, Prof.**
**11 Hankin Street**
**Rehovot(IL)**
Inventor: **Naparstek, Yaakov**
**17 Davidson Street**
**Jerusalem(IL)**

(74) Representative: **Kraus, Walter, Dr. et al**
**Patentanwälte Kraus, Weisert & Partner**
**Thomas-Wimmer-Ring 15**
**D-8000 München 22(DE)**

</td></tr>
</table>

(54) Heparin for the treatment of lupus.

(57) According to the invention there is provided a pharmaceutical composition which inhibits nephritis resulting from human systemic lupus erythematosis (SLE). Such kidney involvement is substantially decreased by administering a dosage of heparin, derivative or biologically effective fragment thereof which is adequate for the desired effect yet which is smaller than a dosage resulting in an appreciable anticoagulant effect.

EP 0 346 810 A2

## FIELD OF THE INVENTION

The invention relates to pharmaceutical compositions for the prevention of nephritis. More particularly, the invention relates to such compositions which are effective in preventing to a large extent kidney involvement in human systemic lupus erythematosis (SLE). According to the invention there is used as active ingredient of such pharmaceutical compositions either a small dosage of heparin, adequate for the desired results yet too small in order to cause an anti-coagulant effect, or of certain derivatives and fragments of heparin, which are effective for the intended purpose, yet which do not cause to any appreciable extent an anticoagulant effect in the patient.

## BACKGROUND OF THE INVENTION

Kidney involvement is a major cause of morbidity and mortality in both human and murine systemic lupus erythematosis (SLE). Nephritis was classically attributed to the formation of circulating anti-DNA - DNA-immune complexes that are passively trapped by the kidney. An alternative mechanism is the direct binding of anti-DNA lupus autoantibodies to structures that are found in normal kidneys, such as heparan sulphate, which is the major glycosaminoglycan constituent of the glomerular basement membrane.

We herein show that heparin and its dervatives cross react with anti-DNA antibodies from human patients and MLR/lpr/lpr mice (a strain of mice that develops spontaneous lupus), and that treatment of these mice with a cross reacting compound such as heparin prevents the development of lupus nephritis.

## SUMMARY OF THE INVENTION

The invention relates to pharmaceutical compositions useful in the prevention of kidney involvement in human systemic lupus erythematosis, in the following, SLE. The compositions of the invention contain an adequate quantity of heparin or of a derivative or fraction of heparin which cross reacts with anti-DNA antibodies from human patients and with MLR/lpr/lpr mice, a strain which spontaneously develops lupus. Such treatments of mice indicates that onset of this disease can be prevented or delayed to a considerable extent. H130 is an anti-DNA antibody (monoclonal) derived from MLR/lpr/lpr mice. This antibody carries the major idiotype of the serum anti-DNA antibodies in these mice and was found to be the major constituent of the immunoglobulins eluted from the MLR/lpr/lpr diseased kidneys. The present invention is based on the finding that both H130 and Immunoglobulins derived from MLR/lpr/lpr kidneys cross react with heparin, with derivatives of heparin and with certain fractions of heparin, and the prolonged application of such derivatives of lupus mice inhibits development of lupus nephritis. This indicates a similar effect with human patients.

Experimental:

Materials

1. Anti-DNA antibodies
   a. The H130 mouse monclonal antibody (Rauch, J.l. et al. J. Immunol. 129:236 (1982)) (donated by R.S. Schwartz, Tufts University, Boston) expresses the major idiotype of the anti-DNA response of the MLR/lpr/lpr mice. High levels of this antibody are in the serum of these animals and even higher levels in immunoglobulins eluted from the diseased kidneys. The serum levels correlate with disease activity. The H130 appears therefore to be a cause of nephritis.
   b. Immunoglobulins eluted directly from the MLR/lpr/lpr kidneys (donated by M. Madaio, Tufts University, Boston).
   c. Serum immunoglobulins from MLR/lpr/lpr mice and (NZB/NZW)F1 mice. Serum antibodies may not cause nephritis.
   d. A-52 - a monoclonal anti-DNA from the (NZB/NZW)F1 (donated by Dr. D. Eilat, Jerusalem).
   e. Immunoglobulins eluted directly from kidneys of human lupus patients.

2. $^3H$ labeled DNA was purchased from New England Nuclear, Boston, Mass.

2

3. Heparin, heparin fragment (AM 71228), Fragmin (84921-51), N/O desulfated heparin, N-acetylated O-desulfated heparin and N-acetylated heparin were received from KabiVitrum, Stockholm.

4 Mice

Female MLR/lp/lpr and (NZB/NZW)F1 mice were purchased from the Jackson Laboratories, Bar-Harbor, Maine.

## Methods

### a. Reaction of antibodies with DNA.

Detection of anti-DNA was performed using the millipore filter assay. Sera, kidney eluates and the monoclonal antibodies H130 and A52 were prepared in serial dilutions in 0.2M borate-saline buffer pH 8.0. Ten $\mu$l of $^3$H-labeled DNA (approximately 6,000 cpm, New England Nuclear, Boston, MA), were added to 100 $\mu$l of the antibody in various dilutions. The mixture was incubated in 37°C for 30 minutes and then for at least 60 minutes at 4°C. The mixture was poured through a vacuum using 0.45 $\mu$m nitrocellulose filters (Milford, Bedford, MA). The filters were washed in 2 aliquots of 3 ml borate buffer and then placed separately in plastic vials. The filters were dried by leaving them at room temperature for at least 16 hours and read with toluene based scintillation fluid in a beta counter.

### b. Cross reactivity of DNA and heparins

The cross reactivity between DNA and heparin was measured using a competitive inhibition assay. Various concentrations of heparin or heparin derivatives were prepared in borate buffer pH 8.0. Fifty $\mu$l of the inhibitor were added to the DNA binding immunoglobulin and the residual binding of the mixture to DNA was measured by a direct binding assay as described.

### c. Heparin treatment

Ten MLR/lpr/lpr mice and ten ((NZB/NZW)F1 mice were treated with daily subcutaneous injections of heparin (5 $\mu$/ml, 0.1 ml). A control group of each strain was treated with daily injections of saline (0.1 ml). The mice were treated from the age of six weeks for 4 months. At that time, mice were sacrificed and their organs examined by light and electron microscopy.

### d. Light microscopy

Tissue samples obtained from lungs, kidneys, lymph nodes, liver and pancreas were fixed in 10% formalin and embedded in paraffin. Six micrometer sections were stained with hematoxylin-eosin, PAS, Masson's trichome and silver stains. Morphological parameters of autoimmune disease were specially evaluated in the kidney. Evaluation of tissue samples was done on a double-blind basis with the code made available only at the end of the entire study.

### e. Electron microscopy

Tissue allocated for electron microscopy was immediately fixed by 4% formaldehyde - 1% glutaraldehyde fixative. Osmification was followed by en block staining with uranyl acetate and dehydration in graded series of ethanol and propylene oxide. Embedding was carried out in Araldite and thin sections were stained with lead citrate. The sections were examined by a Heol 100 CX electron microscopy at 80 KV.

## Results

## 1. Reaction of heparin with anti-DNA antibodies

Heparin inhibited the DNA binding of H130 and of the MLR/lpr/lpr kidney eluate (50% inhibition 0.04 and 10 ug/ml, respectively) (Table 1). It did not inhibit the DNA binding of the MLR/lpr/lpr serum, the (NZB/NZW)F1 serum, or the A52 monoclonal antibody. Heparin was found to react with the H130 anti-DNA antibody with high avidity. This reaction was specific to the H130 antibody as no reaction of heparin with another anti-DNA antibody, the A52 derived from (NZB/NZW)Fl mice was observed. The cross reactivity of heparin and DNA was due in part to the sulfate moieties of heparin. Complete desulfation of the molecule tdtally abolished the cross reactivity, whereas partial desulfation reduced the avidity of its binding to the H130 antibody (not shown).

## 2. The effect of heparin treatment on renal pathology

### i Light microscopy

Saline treated MLR/l mice developed severe glomerulonephritis. Generalized glomerular lesions were found in all cases. The predominant finding was an extensive crescent formation occurring in more than 90% of the glomeruli. These showed capillary loop necrosis often accompanied by hyaline deposits similar to hyaline thrombi described in human SLE. Wire loop-like lesions were a common finding. Scattered glomeruli presented diffuse or segmented proliferation and neotrophilic exudates. Secondary tubular atrophy and dilatation, as well as interstitial round cell inflammatory infiltrates were also noted.

Several medium and small size vessels presented a necrotizing vasculitis. Vasculitis was also found in other organs namely, liver, lung, pancreas and spleen. Patchy round cells, inflammatory infiltrates were present in the liver, lungs, pancreas and heart. MLR/lpr/lpr mice treated with heparin developed only mild forms of glomerulonephritis. In these cases, the findings varied from normal appearing glomeruli to glomeruli showing mesangial hypercellular and increase of mesangial matrix. In the most severely affected glomeruli, capillary wall thickening was often observed. Exudative lesions, as well as crescent formations were rarely found. The tubules were of normal appearance. Besides a scarce round cell inflammatory infiltrate, no changes were found in the interstitium. Vasculitis was absent in all of the treated mice, not only in the kidney, but also in the other organs studied. All (NZB/NZW)F1 mice, whether treated with heparin or with saline, developed severe diffuse proliferation glomerulonephritis.

### ii) Electron microscopy

All the glomeruli from saline treated MLR/lpr/lpr mice that were examined by electron microscopy were found to have numerous electron-dense deposits in the capillary basement membranes. A focal thickening of the basement membrane around the deposits was seen giving the impression of spike formation. Most of the deposits were found in a subepi-thelial location, but subendothelial and intramembranous deposits could also be demonstrated. The mesangial areas were enlarged, containing processes of the proliferating cells and small osmiophylic deposits.

In contrast, glomeruli from heparin treated MLR/lpr/lpr mice were found to have regular and thin capillary basement membranes and subepithelial electron dense deposits were rarely seen. Dense deposits have been found in the mesangium localized mainly in precapillary locations.

The present work shows that lupus nephritis and vasculitis in MLR lupus prone mice can be inhibited by chronic, very low dose heparin therapy. A possible mechanism for the observed effect of heparins is its cross reactivity with the lupus autoantibodies. As was shown in this work, the anti-DNA lupus antibody H130 binds to heparin with a high avidity. This antibody has the public idiotype of the anti-DNA antibodies MLR/lpr/lpr mice, and is found in the eluates of the diseased kidneys. Inhibition of the binding of similar autoantibodies to the kidneys by heparin might explain the inhibitory effect of heparin on the development of nephritis. Thus other compounds which cross react with anti-DNA antibodies and autoantibodies in lupus may be used in a similar manner.

Table 1.

| Inhibition of DNA binding by heparin | | |
|---|---|---|
| | Origin of immunoglobulins | 50% inhibition by heparin ug/ml |
| MLR/lpr/lpr | H130<br>Kidney eluate<br>Serum | 0.04<br>10<br>N.I.* |
| (NZB/NZW)F1 | 52<br>Serum | N.I.<br>N.I. |
| SLE patient #1 | Kidney eluate<br>Serum | 10<br>N.I. |
| SLE patient #2 | Kidney eluate | 25 |
| The immunoglobulins were incubated with heparin in various concentrations and the residual binding to DNA was determined using a filter binding assay. | | |

*N.I. - no inhibition by 100 ug/ml of heparin

Table 2.

| Inhibition of H130 - DNA binding by various heparin derivatives | |
|---|---|
| Heparin derivative* | 50% inhibition of H130 - DNA binding: ug/ml |
| Native heparin<br>Heparin fragment<br>N-acetylated heparin<br>N-acetylated-O-desulfated heparin<br>N/O desulfated heparin | 0.04<br>0.02<br>1.0<br>N.I.**<br>N.I. |

*From Kabivitrum, Stockholm
**N.I. - no inhibition by 10 ug/ml of inhibitor

Table 3.

| Heparin prevents lupus nephritis in MLR/lpr/lpr mice. | | | |
| --- | --- | --- | --- |
| | Kidney | | Kidney |
| | Light microscopy | | Electron microscopy |
| Treatment | Crescents | Vasculitis | Subepithelia & subendothelial dense deposits |
| Saline | + | + | + |
| Heparin | - | - | - |
| The MLR/lpr/lpr mice were treated with daily subcutaneous injections of either saline or heparin (500 U/day). After four months the mice were sacrificed and their kidneys examined by light and electron microscopy. | | | |

Claims

1. A pharmaceutical composition for the inhibition of nephritis associated with anti-DNA antibodies in SLE which comprises an effective quantity of heparin or a derivative or a fraction or fragment of same, which compound is used in a quantity not substantially affecting the blood coagulation of the human treated.

2. A composition according to claim 1, where the heparin is used in a dosage not exceeding about one tenth that of the dosage used in anti-coagulation treatment.

3. A pharmaceutical composition according to claim 1 where the compound is selected from N-acetylated heparin, or fragments thereof.

4. A composition according to claim 1, where the compound used is an effective fragment of heparin.

5. Compositions for the prevention of SLE associated nephritis, or for delaying its onset, comprising heparin or a fragment or derivative thereof, substantially as hereinbefore described.

6. A composition according to claim 1 where the compound used is a high molecular weight fraction or derivative of heparin, or heparin devoid of low molecular weight components.